# EUROPEAN PATENT APPLICATION

(11) **EP 4 781 963 A1**
(43) Date of publication of application: **29.07.2026**
(21) Application number: 26154376.3
(22) Date of filing: 27.01.2026
(51) Int. Cl.: A61G 7/012, A61G 7/057, G16H 40/63, A61G 7/05

(54) **IMPROVEMENTS FOR MEDICAL DEVICE OPERATIONS**

(30) Priority: 28.01.2025 US 202563750396 P
(71) Applicant: Hill-Rom Services, Inc., Batesville, IN 47006-9167 (US)
(72) Inventor: CHRISTIE, John D., Batesville, 47006-9167 (US); MOK, Qinglin, Batesville, 47006-9167 (US)
(74) Representative: Reddie & Grose LLP

(57) **Abstract**

A patient support apparatus includes a frame and a deck coupled to the frame. The deck is movable relative to the frame and is configured to support a mattress. At least one actuator moves at least one of the mattress and the deck to carry out a therapy or caregiver assistance function. At least one sensor detects movement of a patient supported on the mattress. The patient support apparatus detects a state of actuation of the at least one actuator, determines a type of movement of the patient based on one or more outputs of the at least one sensor, and identifies an actuation feature of the at least one actuator associated with the type of movement of the patient. The patient support apparatus recommends information about utilizing the actuation feature when the actuation feature is not utilized when the type of movement of the patient occurred.

## Description

Medical device training is crucial for ensuring safe and effective use of healthcare technologies. Proper training helps healthcare professionals understand how to operate devices correctly, reducing the risk of errors that could harm patients. It also ensures compliance with regulatory standards and improves overall patient outcomes by promoting efficiency for the provision of healthcare. Additionally, continuous training is essential as medical devices evolve, ensuring that staff remain up-to-date on the latest features and safety protocols. Ultimately, well-trained personnel contribute to safer healthcare environments and enhanced patient care.

In general terms, the present disclosure relates to monitoring interactions with medical devices for improving the training of the devices. In one possible configuration, training is recommended for a feature of a medical device when a state of the medical device indicates the feature was not used when an event occurred. Various aspects are described in this disclosure, which include, but are not limited to, the following aspects.

One aspect relates to a patient support apparatus, comprising: a frame; a deck coupled to the frame, the deck being movable relative to the frame, and the deck being configured to support a mattress; at least one actuator configured to move at least one of the mattress and the deck to perform a therapy or caregiver assistance function; at least one sensor configured to detect movement of a patient supported on the mattress; at least one processing device; and at least one memory device storing software instructions that, when executed by the at least one processing device, cause the at least one processing device to: detect a state of actuation of the at least one actuator; determine a type of movement of the patient based on one or more outputs of the at least one sensor; identify an actuation feature of the at least one actuator that is associated with the type of movement of the patient; determine whether the actuation feature is utilized when the type of movement of the patient occurred based on the state of actuation of the at least one actuator; and recommend information about utilizing the actuation feature when the actuation feature is not utilized when the type of movement of the patient occurred.

Another aspect relates to a system for operating a medical device, the system comprising: at least one processing device; and at least one memory device storing software instructions that, when executed by the at least one processing device, cause the at least one processing device to: determine a state of the medical device; detect an event that occurs during the state of the medical device; identify a feature of the medical device that is associated with the event; and display information regarding a recommendation about utilizing the feature when the state of the medical device indicates the feature was not used when the event occurred.

Another aspect relates to a method for operating a medical device, the method comprising: determining a state of the medical device; detecting an event that occurs during the state of the medical device; identifying a feature of the medical device that is associated with the event; and displaying training for the feature when the state of the medical device indicates the feature was not used when the event occurred.

The disclosure provides a practical application that improves patient care through technological integration of sensors, actuators, artificial intelligence, and automated responses in a hospital bed. This practical implementation addresses technological problems in healthcare while providing concrete benefits and improvements to existing patient care systems.

The preferred disclosed patient support apparatus implements a concrete technological solution by utilizing multiple integrated sensors to capture real-time patient data, including load cells that detect patient movement and weight distribution, pressure sensors that detect pressure inside mattress bladders, and position sensors that monitor bed articulation angles. This sensor fusion creates a comprehensive monitoring system that enables detection of patient behaviors and conditions.

In at least preferred embodiment, this provides a tangible improvement to healthcare technology through artificial intelligence that processes the sensor data to engage users in a meaningful way to improve utilization of the patient support apparatus. This is a specific technological implementation that displays information regarding a recommendation about utilizing a particular feature of the patient support apparatus when the state of a patient support apparatus indicates the feature was not used when a particular event occurred.

The practical application extends to specific automated responses and adjustments. When the system detects patient conditions requiring intervention, the system can automatically adjust bed settings, such as repositioning articulated sections of the bed or modifying mattress pressure, while following defined protocols and safety parameters. These automated responses represent concrete physical actions that directly improve patient care and comfort.

In preferred embodiments, this has practical application through integration with existing healthcare systems. For example, the technology described herein interfaces with electronic health records (EHR) systems, admission/discharge/transfer (ADT) systems, and nurse call systems, creating a comprehensive care platform that enhances healthcare delivery through specific technological improvements. This integration enables the system to make informed decisions based on patient medical history and current care protocols.

The system includes specific display implementations that provide visual feedback through mounted display screens on the siderails of the patient support apparatus. These displays show recommendations and summaries of patient and bed interactions, providing users with actionable information that improves operational efficiency by the patient support apparatus. The visual interface components displayed on the patient support apparatus represent specific technological implementations that provide practical, usable tools for healthcare providers.

The practical application is further demonstrated through the system's ability to generate specific alerts and recommendations based on detected feature usage or lack thereof. These alerts are communicated through defined channels to user devices, representing a concrete implementation of the technology that can improve patient care outcomes. The patient support apparatus has the ability to learn from user interactions and patient outcomes, which demonstrates a practical application that continuously improves the effectiveness of the patient support apparatus through specific technological means.

A variety of additional aspects will be set forth in the description that follows. The aspects can relate to individual features and to combination of features. It is to be understood that both the foregoing general description and the following detailed description are exemplary and explanatory only and are not restrictive of the broad inventive concepts upon which the embodiments disclosed herein are based.

The invention will now be further described by way of example with reference to the accompanying drawings, in which:
FIG. 1 is an isometric view of an example of a patient support apparatus.
FIG. 2 is a side view of another example of the patient support apparatus of FIG. 1 shown in a lower position.
FIG. 3 is another side view of the patient support apparatus of FIG. 1 shown in an upper position.
FIG. 4 is another side view of the patient support apparatus of FIG. 1 shown in a Trendelenburg position.
FIG. 5 is another side view of the patient support apparatus of FIG. 1 shown in a reverse Trendelenburg position.
FIG. 6 is an isometric view of another example of the patient support apparatus of FIG. 1 with a deck of the patient support apparatus drawn in phantom.
FIG. 7 schematically illustrates an example of a system for providing medical device training for the patient support apparatus of FIG. 1.
FIG. 8 schematically illustrates an example of a method of providing medical device training that can be performed by the patient support apparatus of FIG. 1.
FIG. 9 illustrates examples of features of the patient support apparatus of FIG. 1 that can be used when providing care to a patient occupying the patient support apparatus.
FIG. 10 illustrates an example of a display screen of the patient support apparatus of FIG. 1 that includes a message displayed in accordance with the method of FIG. 8.

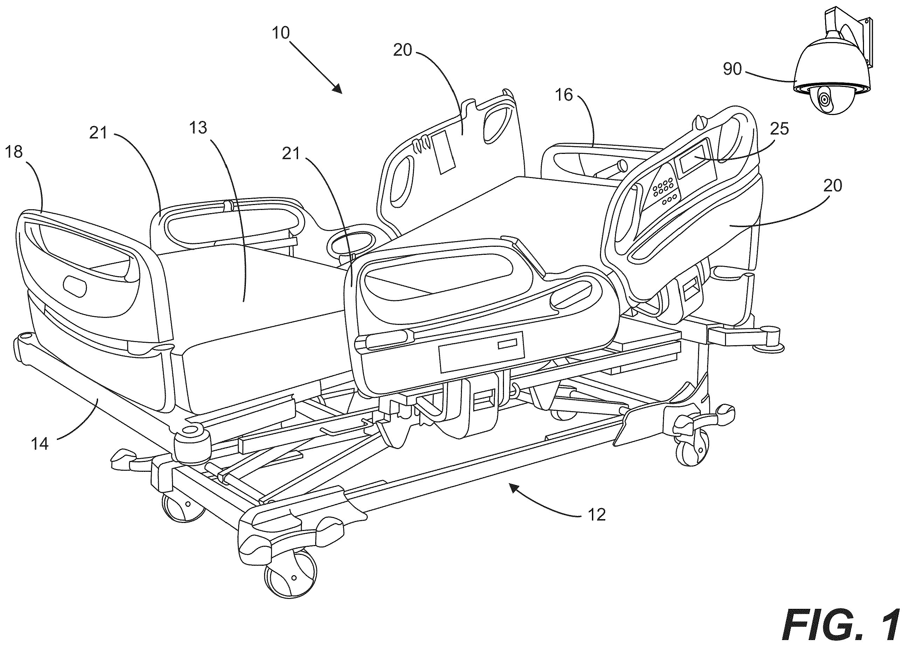

FIG. 1 is an isometric view of an example of a patient support apparatus 10. The patient support apparatus 10 is configured to monitor interactions by users to determine whether additional training would improve the effectiveness and efficiency of using the patient support apparatus 10. In some instances, artificial intelligence is used to detect interactions and/or patterns of interactions with the patient support apparatus 10, and the artificial intelligence recommends training to improve the efficacy of the patient support apparatus 10.

While the following description refers to the patient support apparatus 10, the concepts described herein can be used to improve the effectiveness and efficiency of additional types of medical devices such as other types of patient support apparatuses and surfaces, patient monitoring devices, vision screening devices, diagnostic cardiology devices, and infusion pumps.

In the example shown in FIG. 1, the patient support apparatus 10 is depicted as a hospital bed. In alternative examples, the patient support apparatus 10 can include a stretcher, a surgical table, or other structures configured to support a patient within a healthcare environment such as a hospital, nursing home, long term care facility, and the like.

The patient support apparatus 10 includes a frame 12, a deck 14 coupled to the frame 12, and a mattress 13 positioned on the deck 14. The patient support apparatus 10 includes a headboard 16 coupled to frame 12 and a footboard 18 coupled to deck 14. The frame 12 is configured to raise and lower the deck 14 relative to the floor and to move deck 14 in various positions such as the Trendelenburg position and the reverse Trendelenburg position.

The patient support apparatus 10 further includes a pair of upper siderails 20 and a pair of lower siderails 21 coupled to the frame 12. In the example shown in FIG. 1, an upper siderail 20 on the right side of the patient support apparatus 10 includes a display screen 25. In alternative examples, the display screen 25 can be positioned elsewhere on the patient support apparatus 10. The display screen 25 can include a touchscreen that receives inputs from users such as physicians, registered nurses (RN), and other healthcare professionals. The display screen 25 can use various technologies to sense the inputs such as capacitance to detect changes in an electrical field when a conductive object (e.g., finger or stylus) touches the screen.

As further shown in FIG. 1, a camera 90 captures visual data of the users of the patient support apparatus 10. The visual data can include images, videos, and the like. In some examples, the camera 90 includes a depth camera that uses infrared or structural light to measure 3D position. In some examples, the camera 90 is a pan-tilt-zoom (PTZ) camera that includes mechanical parts that allow the camera 90 to swivel left to right, tilt up and down, and zoom in and out for capturing the visual data of the users of the patient support apparatus 10. In some examples, the camera 90 can capture the visual data under different wavelengths of light such that the camera 90 can capture color images/videos, infrared images/videos, and the like.

The visual data captured by the camera 90 can be analyzed to identify interactions that the users make with the patient support apparatus 10. The visual data captured by the camera 90 can also be analyzed to detect events such as when the patient is pulled up while resting on the patient support apparatus 10, when the patient is turned on the patient support apparatus 10, when the patient exits the patient support apparatus 10, and other events. In some examples, the visual data captured by the camera 90 is analyzed using artificial intelligence.

Additional types of sensors can be deployed to detect and/or identify interactions by users of the patient support apparatus 10 such as ultra-wideband (UWB) anchors that track movements by detecting how signals are reflected off the body; infrared sensors that detect the body heat and movement; light detection and ranging (LIDAR) sensors that scan laser pulses for mapping positions of persons and objects; ultrasonic sensors that emit high frequency sound waves and detect reflections from persons and objects; Wi-Fi sensors that monitor disturbances in Wi-F- signals caused by movements of persons and objects, and additional types of sensors.

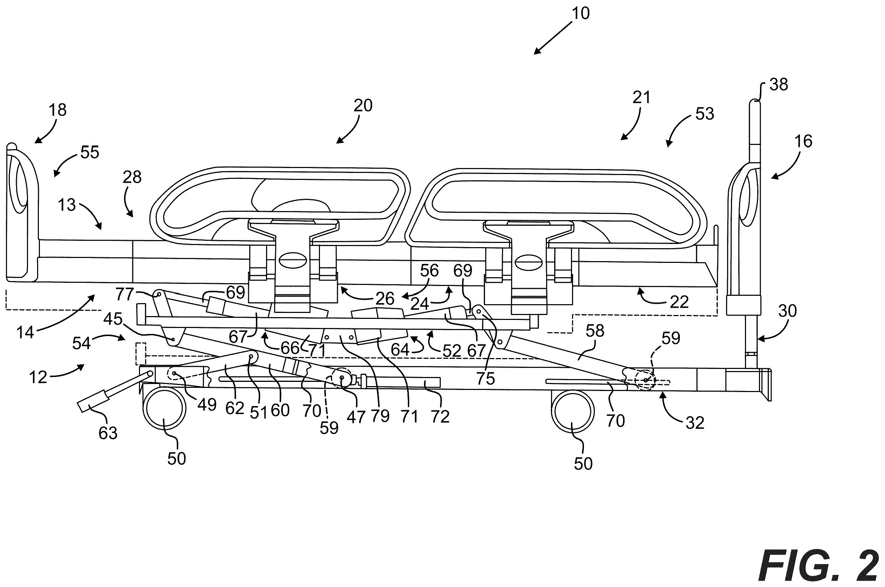

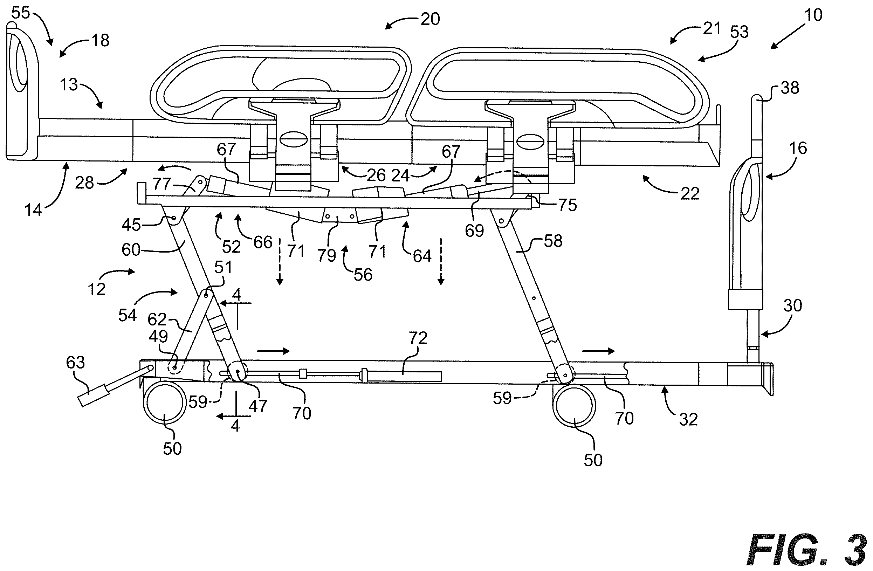

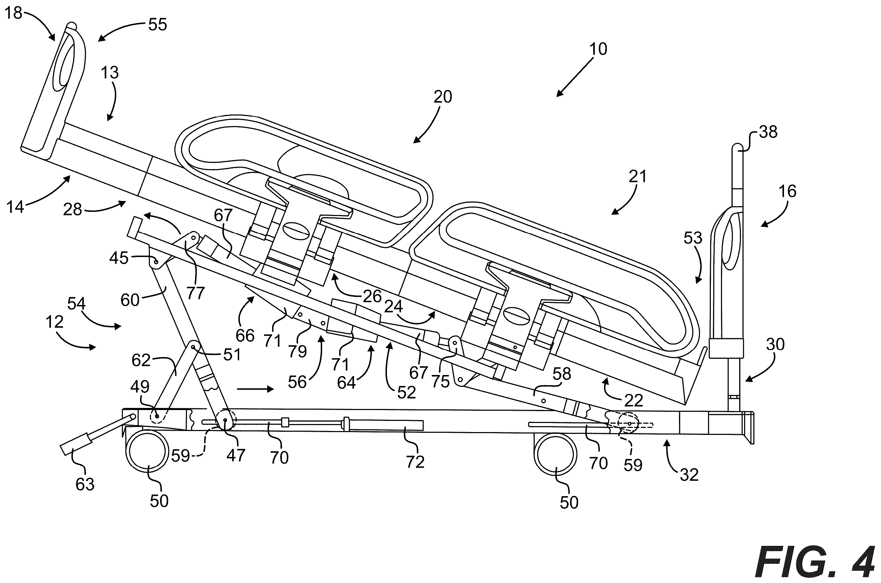

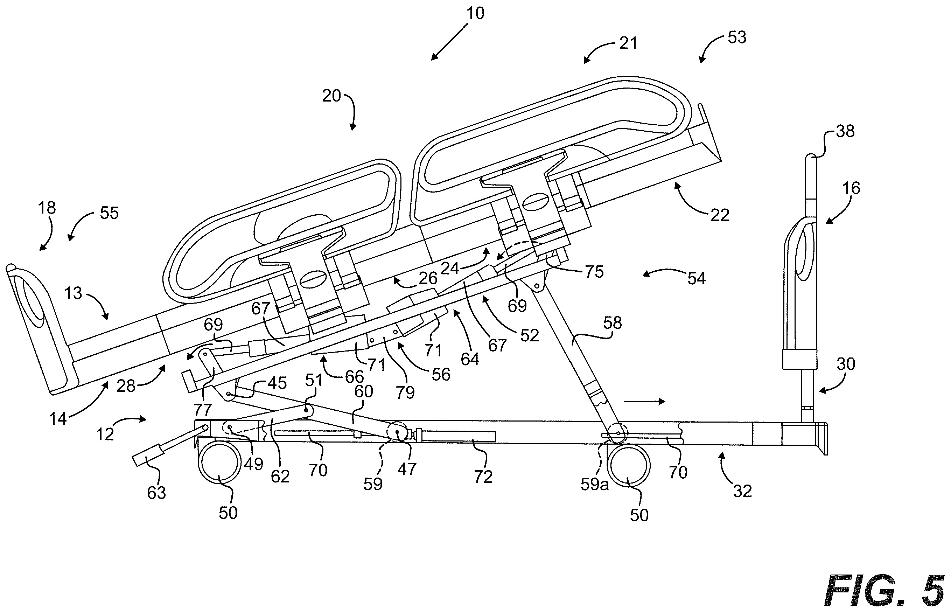

FIG. 2 is a side view of another example of a patient support apparatus 10 shown in a lower position. FIG. 3 is another side view of the patient support apparatus 10 shown in an upper position. FIG. 4 is another side view of the patient support apparatus 10 shown in a Trendelenburg position. FIG. 5 is another side view of the patient support apparatus 10 shown in a reverse Trendelenburg position. Referring now to FIGS. 2-5, the deck 14 has a head section 22, a back section 24, a seat section 26, and a foot section 28. In some examples, the foot section 28 of the deck 14 is extendable. The foot section 28, the seat section 26, the back section 24, and the head section 22 of the deck 14 can each be individually tilted relative to the ground to adjust the contour of a support surface such as the mattress 13 for patient comfort.

The headboard 16 includes a base 30 coupled to the frame 12. The headboard 16 includes handles 38 for pushing the patient support apparatus 10 about a healthcare facility. The frame 12 includes a base member 32, and a plurality of casters 50 coupled to the base member 32 to permit the patient support apparatus 10 to be rolled about a healthcare facility.

The patient support apparatus 10 includes a linkage system 54 that couples the base member 32 to an intermediate member 52 to permit relative motion therebetween. The patient support apparatus 10 further includes an actuator system 56 that provides power to actuate the linkage system 54 to move the intermediate member 52 relative to base member 32 causing the frame 12 of the patient support apparatus 10 to change position. The actuator system 56 includes at least one actuator configured to move at least one of the mattress 13 and the deck 14 to carry out a therapy or caregiver assistance function, as discussed in more detail further below.

The linkage system 54 includes a pair of head links 58 that are pivotably coupled to the intermediate member 52 at a head end 53 of the patient support apparatus 10. The head links 58 are slidably coupled to the base member 32. The patient support apparatus 10 further includes a pair of foot links 60 that are pivotably coupled to the intermediate member 52 at a foot end 55 of the patient support apparatus 10. The foot links 60 are slidably coupled to the base member 32. A pair of guide links 62 are further pivotably coupled to the base member 32 at pivot points 49. The pair of guide links 62 are pivotably coupled to respective foot links 60 at pivot points 51.

The linkage system 54 includes rollers 59 rotatably coupled to the lower ends of the head and foot links 58, 60. The rollers 59 are positioned inside slots 70 formed in inner side walls of the base member 32. The rollers 59 ride over a lower wall of the base member 32 to provide a smooth rolling movement between the head and foot links 58, 60 and the base member 32 to facilitate the sliding movement of the head and foot links 58, 60 in the base member 32.

The patient support apparatus 10 further includes a caster braking system that includes a caster-brake link extending through the base member 32 adjacent to the roller 59. The caster braking system interconnects each caster 50 to provide simultaneous braking of casters 50. When the caregiver steps on at least one of the foot brake pedals 63, the caster braking system causes the casters 50 to lock against rolling.

The guide links 62 restrict the motion of foot links 60 such that the pivot point between foot links 60 and the intermediate member 52 is restrained to move vertically without moving horizontally. This restriction prevents horizontal movement of the intermediate member 52 relative to the base member 32 during the raising and lowering of the intermediate member 52. This restrained movement prevents the intermediate member 52 from moving through an arch while moving between the upper and lower positions so that the intermediate member 52 can be raised and lowered without requiring additional hospital room for clearance.

Each of the guide links 62 is pivotably coupled to a foot link 60 at a pivot point 51 and is pivotably coupled to the base member 32 at a pivot point 49. The distance between pivot points 49, 51 of the guide links 62 is one half the distance between pivot points 47 of the rollers 59 and pivot points 45 at upper ends of the foot links 60. The distance between pivot point 51 of each guide link 62 and the pivot point 49 at the lower end of each guide link 62 is equal to the distance between the pivot point 51 of each guide link 62 and the pivot point 45 of each foot link 60. The link geometry maintains the pivot points 45 at the upper ends of the foot links 60 in vertical alignment with the pivot point 49 at the lower end of the guide links 62 when raising and lowering the intermediate member 52 relative to the base member 32 of the frame 12.

The actuator system 56 provides the force and power necessary to raise and lower the intermediate member 52. The actuator system 56 includes a head link actuator 64 coupled to the head links 58 and the intermediate member 52 and a foot link actuator 66 coupled to the foot links 60 and the intermediate member 52. The head link actuator 64 is coupled to the head links 58 through a link 75 coupled to a cross strut which extends between and is rigidly coupled to each of the head links 58. The foot link actuator 66 is coupled to the foot links 60 through a link 77 coupled to a cross strut which extends between and is rigidly coupled to each of the foot links 60. The cross strut coupled to the head links 58 coordinates the simultaneous movement thereof, whereas the cross strut coupled to the foot links 60 coordinates simultaneous movement thereof.

The head and foot link actuators 64, 66 have expandable lengths to adjust the angular position of the head and foot links 58, 60 relative to the intermediate member 52 such that the head end 53 and the foot end 55 of the intermediate member 52 can be raised or lowered relative to each other. Each of the head and foot link actuators 64, 66 can have an electric linear actuator having respective cylinder bodies 67, cylinder rods 69, and motors 71 that operate to extend and retract the cylinder rods 69 relative to the cylinder bodies 67. The cylinder rods 69 are each pivotably coupled to respective links 75, 77 and the motors 71 are each pivotably coupled to a bracket 79 included in the intermediate member 52 as shown, for example, in FIG. 2.

When the head and foot link actuators 64, 66 are actuated simultaneously, such that one of the head and foot link actuators 64, 66 extends while the other of the head and foot link actuator 66, 64 retracts, the intermediate member 52 either raises away from or lowers toward the base member 32 such that the intermediate member 52 is maintained in a horizontal position and does not swing outwardly or inwardly relative to the base member 32.

When the head link actuator 64 is activated and the foot link actuator 66 is maintained at a constant length, the intermediate member 52 moves to the Trendelenburg position shown in FIG. 4 such that the head end 53 of the intermediate member 52 is lowered and the foot end 55 of the intermediate member 52 is raised. When the foot link actuator 66 is activated and the head link actuator 64 is maintained at a constant length, the intermediate member 52 moves to the reverse Trendelenburg position such that the foot end 55 of intermediate member 52 is lowered and the head end 53 of intermediate member 52 is raised, as is shown in FIG. 5.

As shown in FIGS. 2 and 3, the deck 14 is lowered by activating both the head and foot link actuators 64, 66. As the length of the foot link actuator 66 increases, the angle between the foot links 60 and the intermediate member 52 decreases and the foot end 55 of the intermediate member 52 lowers. As the length of the head link actuator 64 decreases, the angle between the head links 58 and the intermediate member 52 increases and the head end 53 of the intermediate member 52 lowers as shown, for example, in FIG. 2.

As the length of the foot link actuator 66 continues to increase and the length of the head link actuator 64 continues to decrease, the intermediate member 52 continues to lower from the upper position of FIG. 3 to the lower position shown in FIG. 2. Because the head and foot link actuators 64, 66 decrease and increase their respective lengths at substantially the same rate, the intermediate member 52 remains substantially horizontal while moving from the upper position of FIG. 3 to the lower position of FIG. 2. To position the intermediate member 52 in the upper position, the head link actuator 64 is lengthened and the foot link actuator 66 is shortened until each of the head and foot link actuators 64, 66 returns to the length as shown in FIG. 3.

The linkage system 54 and the actuator system 56 also cooperate to move intermediate member 52 to the Trendelenburg position shown in FIG. 4. To move the intermediate member 52 to the Trendelenburg position, the head link actuator 64 decreases its length such that the angle between the intermediate member 52 and the head links 58 increases. The head end 53 of the intermediate member 52 lowers and the length of the foot link actuator 66 remains substantially constant to provide a pivot point about which the intermediate member 52 rotates. As the intermediate member 52 rotates, the foot end 55 of the intermediate member 52 is raised. To reposition the intermediate member 52 into the upper horizontal position, the length of the head link actuator 64 is increased until returning to the previous length.

The actuator system 56 and the linkage system 54 also cooperate to position the intermediate member 52 into the reverse Trendelenburg position as shown in FIG. 5. To move the intermediate member 52 to the reverse Trendelenburg position, the length of the foot link actuator 66 is increased such that the angle between the foot links 60 and the intermediate member 52 is decreased and the foot end 55 of the intermediate member 52 lowers. The length of the head link actuator 64 remains substantially constant such that the intermediate member 52 pivots about the head links 58. As the intermediate member 52 pivots, the head end 53 of the intermediate member 52 is raised as the foot end 55 of the intermediate member 52 lowers. To reposition the intermediate member 52 in the upper horizontal position, the length of the foot link actuator 66 is decreased until it is returned to its previous length.

The patient support apparatus 10 includes dampers 72 coupled to the inner walls of the base member 32 to engage the lower ends of the foot links 60. The dampers 72 aid in raising the intermediate member 52 and the deck 14 from the lower and reverse Trendelenburg positions. When lowering the foot end 55 of the intermediate member 52, the dampers 72 resist movement of the foot links 60 and store potential energy as a result of the lowering of the foot end 55 of the intermediate member 52. For example, as the foot links 60 move along the slots 70, the dampers 72 are compressed such that potential energy is stored.

As the intermediate member 52 is moved from the lower position of FIG. 2, to the upper position of FIG. 3, the dampers 72 aid the foot link actuators 66 in raising the foot end 55 of the intermediate member 52 by pushing the lower ends of the foot links 60 in the direction that raises the foot end 55 of the intermediate member 52 to the upper position. Because the dampers 72 store potential energy during the lowering of the foot end 55 of the intermediate member 52, the foot link actuator 66 does not need to be as powerful to raise the foot end 55 of the intermediate member 52 from the lower position to the upper position. In alternative examples, a more powerful foot link actuator can be provided such that the dampers are not provided.

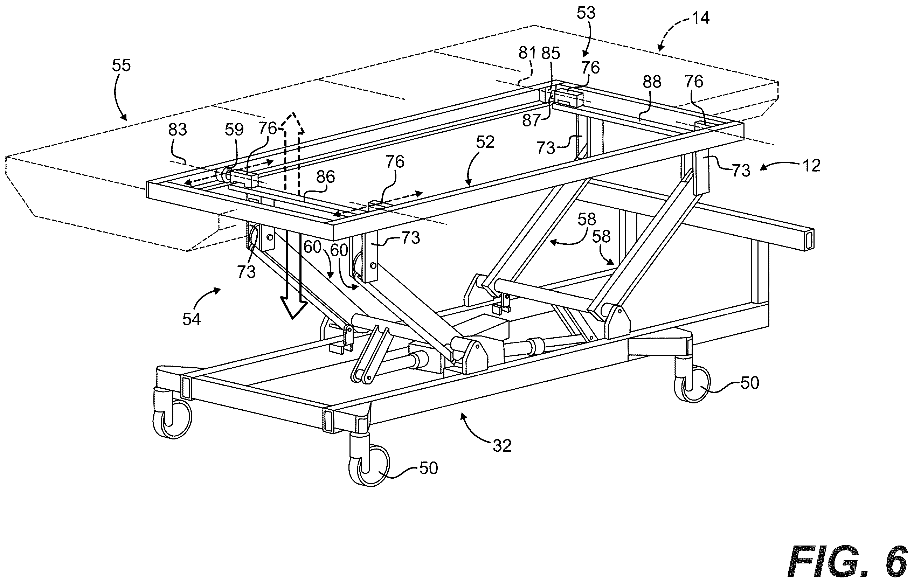

FIG. 6 is an isometric view of another example of the patient support apparatus 10 with the deck 14 drawn in phantom. Load cells 76 are positioned on the frame 12 such as on the intermediate member 52. As shown in FIG. 6, a load cell 76 is positioned between respective blocks 85 and rollers 59 and third links 73. The load cells 76 measure the respective weight applied to each third link 73. The third links 73 remain vertical such that no trigonometric calculations need to be made to correct the weight measurement due to the orientation of the load cell 76 relative to the floor. The load cells 76 are rigidly coupled to respective struts 86, 88.

Since the third links 73 remain substantially vertical, the load cells 76 also remain in a substantially vertical orientation simplifying the overall calculation necessary for determining the weight of the patient. To determine the total weight of the patient, the weights measured by the load cells 76 are totaled and the predetermined weight of the components of the patient support apparatus 10 supported by the load cells 76 are subtracted from this total resulting in the weight of the patient. The weights measured from the load cells 76 do not need to be adjusted for the angular position of intermediate member 52 because the load cells 76 remain vertically oriented.

The pair of blocks 85 are fixed to the intermediate member 52 adjacent to the head end 53 and the load cells 76 associated with the head links 58 each include a stud 87 extending transversely therefrom into a bore formed in the respective block 85. As the intermediate member 52 tilts relative to the base member 32, the blocks 85 tilt along with the intermediate member while pivoting relative to the load cells 76 on the stud 87 about a pivot axis 81. As the intermediate member 52 tilts relative to the base member 32, the rollers 59 rotate about the pivot axis 83 relative to the load cells 76 while also rolling either toward or away from blocks 85 depending upon the direction that intermediate member 52 tilts.

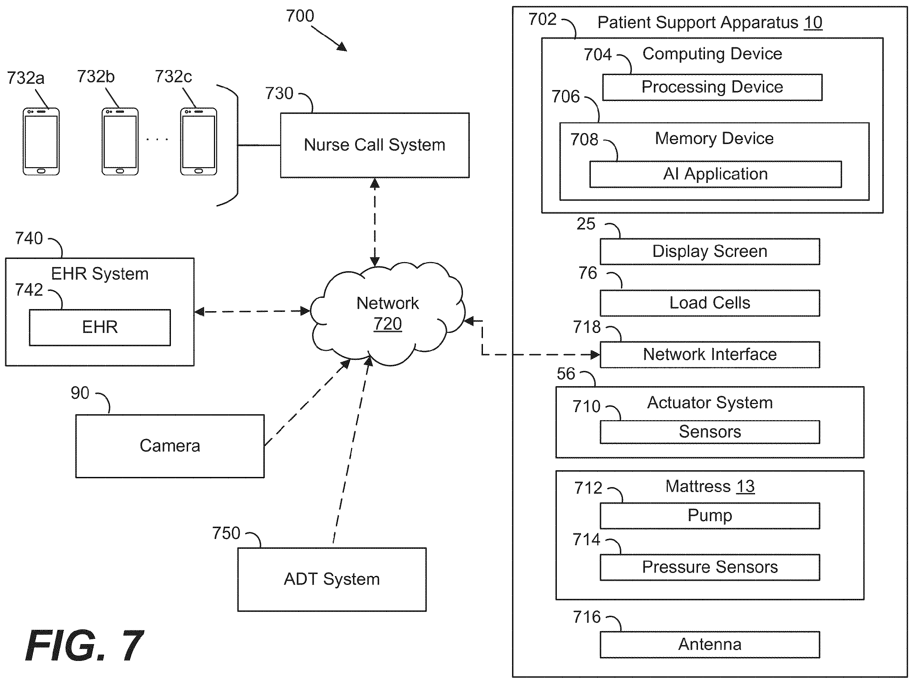

FIG. 7 schematically illustrates an example of a system 700 for providing medical device training for the patient support apparatus 10. In this illustrative example, the patient support apparatus 10 is communicatively connected via a network 720 to a nurse call system 730, an electronic health record (EHR) system 740, the camera 90, and an admission, discharge, and transfer (ADT) system 750. In alternative examples, the patient support apparatus 10 can directly connect (i.e., without using the network 720) to one or more of the nurse call system 730, the EHR system 740, the camera 90, and the ADT system 750.

In the example embodiment shown in FIG. 7, the patient support apparatus 10 is connected to the camera 90 and to other systems and devices that capture data for detecting an interaction by a user with the patient support apparatus 10. Based on the interaction, the patient support apparatus 10 determines whether additional user training would be advantageous to use the patient support apparatus 10 more effectively in the provision of healthcare. As will be described in more detail further below, in some instances, artificial intelligence is used to detect the interaction and to recommend the training based on the detection of the interaction.

As shown in FIG. 7, the patient support apparatus 10 includes a computing device 702 having at least one processing device 704 and at least one memory device 706 that stores software instructions that, when executed by the at least one processing device 704, cause the at least one processing device 704 to perform the various aspects, functions, and operations described herein. The at least one processing device 704 is an example of a processing unit such as a central processing unit (CPU). The at least one processing device 704 can include one or more CPUs. The at least one processing device 704 can also include one or more digital signal processors, field-programmable gate arrays, and/or other types of electronic circuits.

The at least one memory device 706 is an example of a computer-readable data storage device that operates to store data and instructions for execution by the at least one processing device 704. The at least one memory device 706 stores an artificial intelligence (AI) application 708 that monitors usage of the patient support apparatus 10, and determines whether the patient support apparatus 10 can be more effectively used to provide healthcare. In some examples, the Al application 708 uses machine learning to detect interactions or patterns of interactions by users of the patient support apparatus 10, which to recommend training for the patient support apparatus 10 based on the detected interactions or patterns of interactions.

As an example, the AI application 708 can detect an interaction with the patient support apparatus 10, and determine that a feature of the patient support apparatus 10 was not used during the interaction. As a result, the Al application 708 generates a recommendation for training to improve the effectiveness of using the patient support apparatus 10.

The recommendation for training can be displayed on the display screen 25 of the patient support apparatus 10. Additionally, or alternatively, the recommendation for training is routed by the nurse call system 730 to a device 732 of the user of the patient support apparatus 10 such as a mobile smartphone, a tablet computer, a workstation computer, and the like.

In further examples, patterns of interactions with the patient support apparatus 10 are detected by the AI application 708, and the recommendation for training is based on the patterns of interactions. In such examples, the recommendation for training can be for an entire team, unit, department, or other group of users within the healthcare facility. In some examples, the recommendation for training can be distributed by the nurse call system 730 to a plurality of the devices 732 for training a plurality of the users of the patient support apparatus 10.

In some examples, the Al application 708 utilizes artificial intelligence to detect interactions and/or patterns of interactions with the patient support apparatus 10 based on data from the load cells 76 of the frame 12 and/or the data from pressure sensors 714 of the mattress 13. For example, artificial intelligence such as machine learning is used to detect that a patient is being pulled up on the mattress 13 by a caregiver based on the data from the load cells 76 of the frame 12 and/or the data from the pressure sensors 714 of the mattress 13.

As another example, artificial intelligence such as machine learning is used to detect that a patient is being turned on the mattress 13 by a caregiver based on the data from the load cells 76 of the frame 12 and/or the data from the pressure sensors 714 of the mattress 13.

As another example, artificial intelligence such as machine learning is used to detect that a patient is being laterally transferred from the patient support apparatus 10 to another patient support apparatus or other type of support surface based on the data from the load cells 76 of the frame 12 and/or the data from the pressure sensors 714 of the mattress 13.

As another example, artificial intelligence such as machine learning is used to detect that a caregiver is helping the patient exit the patient support apparatus 10 based on the data from the load cells 76 of the frame 12 and/or the data from the pressure sensors 714 of the mattress 13.

The Al application 708 can include a machine learning model built from data that is collected in a controlled environment and labeled such as data from the sensors on the patient support apparatus 10; the sensors proximate to the patient support apparatus 10 such as the camera 90; data acquired from user inputs on a control panel of the patient support apparatus 10 such as user inputs on the touchscreen or buttons/switches adjacent to the display screen 25; and/or data acquired from the EHR system 740.

The data is cleaned and normalized. Feature extraction is executed on the data to identify key characteristics or patterns. Supervised learning can be used to determine whether regression algorithms like linear regression, random forest, or neural networks generate predictions that have a highest accuracy, or whether classification algorithms like support vector machines or decision trees generate predictions that have a highest accuracy. Once the machine learning model is trained and evaluated, the machine learning model can be installed on the patient support apparatus 10 to analyze new data in real-time.

The training of the AI application 708 can leverage existing tools from MathWorks and Python, with the Al application 708 being trained using proprietary data belonging to a manufacturer of the patient support apparatus 10. While the AI application 708 is designed to potentially run directly on the patient support apparatus 10, current hardware limitations may cause the Al application 708 to be performed on external servers until hardware capabilities of the patient support apparatus 10 are enhanced.

The training architecture incorporates multiple layers of data integration and processing. The Al application 708 learns from various sensor inputs including load cell data that tracks patient movement and weight distribution, pressure sensor readings from mattress bladders, and positional data from bed angle sensors. This sensor fusion approach enables comprehensive monitoring of patient behavior and status.

The training methodology implements sophisticated behavioral pattern recognition by processing historical data from both individual patient interactions and larger patient populations. This includes analyzing the outcomes of previous recommendations and correlating patient conditions with specific bed settings. The Al application 708 employs closed-loop learning to continuously improve performance based on the success rates of its recommendations.

Contextual awareness can be built into the training process through the integration of multiple healthcare data sources. The Al application 708 learns to incorporate patient medical history from electronic health records, admission and discharge data, and nurse call system interactions. This comprehensive data integration enables the Al application 708 to make more informed and contextually appropriate recommendations.

The neural network architecture is carefully designed with consideration given to various training techniques specifically chosen for medical applications. For example, large language models (LLMs) can be used to implement generative Al that is optimized and adapted for this specific healthcare application. The training process is deliberately controlled and targeted, recognizing the stringent requirements of medical device applications.

The at least one memory device 706 includes computer-readable media, which includes any media that can be accessed by the at least one processing device 704. The computer-readable media can include computer-readable storage media and computer-readable communication media. The computer-readable storage media includes volatile and nonvolatile, removable and non-removable media implemented in any device that can store information such as computer-readable instructions, data structures, program modules, or other data. The computer-readable storage media can include random access memory, read only memory, electrically erasable programmable read only memory, flash memory, and other memory technology, including any medium that can be used to store information that can be accessed by the at least one processing device 704. The computer-readable storage media is non-transitory.

The computer-readable communication media embodies computer-readable instructions, data structures, program modules or other data in a modulated data signal such as a carrier wave or other transport mechanism and includes any information delivery media. The term modulated data signal refers to a signal that has one or more of its characteristics set or changed in such a manner as to encode information in the signal. The computer-readable communication media can include wired media such as a wired network or direct-wired connection, and wireless media such as acoustic, radio frequency, infrared, and other wireless media. Combinations of any of the above are within the scope of computer-readable media.

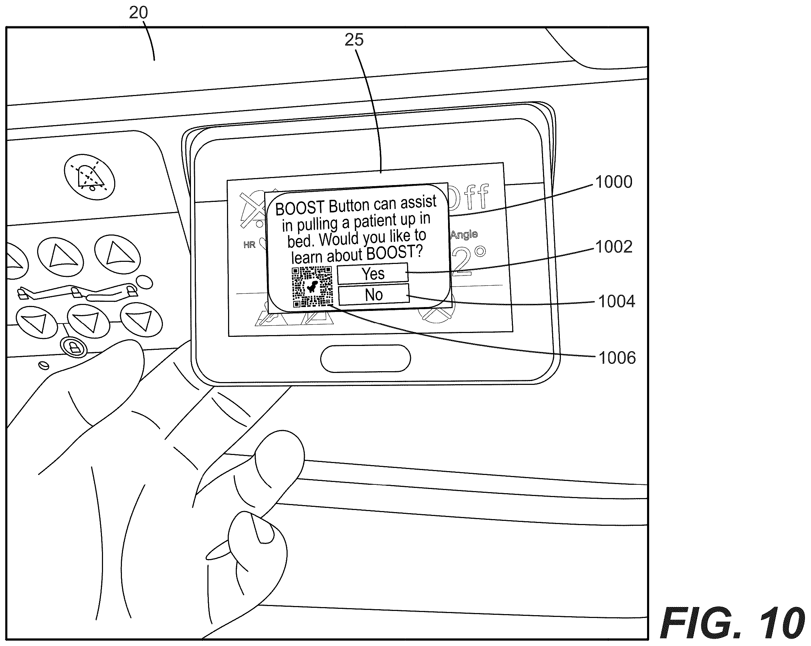

The patient support apparatus 10 includes the display screen 25 which is communicatively connected to the computing device 702. The display screen 25 can include a touchscreen. When the display screen 25 detects an input on the touchscreen, the display screen 25 sends data to the at least one processing device 704, which processes the input for generating a response. An example of the display screen 25 is illustrated in more detail in FIG. 10.

The patient support apparatus 10 further includes the actuator system 56 which is communicatively connected to the computing device 702. The actuator system 56 includes one or more sensors 710 that detect the position of the frame 12, including the relative positioning of the deck 14, the base member 32, and the intermediate member 52 which can be communicated to the computing device 702 for determining a state of the patient support apparatus 10 such as whether the patient support apparatus 10 is in the lower position, the upper position, the Trendelenburg position, or the reverse Trendelenburg position. Also, the computing device 702 communicates instructions to the actuator system 56 to position the frame 12 into the lower position, the upper position, the Trendelenburg position, or the reverse Trendelenburg position such as based on commands received from the touchscreen of the display screen 25.

The one or more sensors 710 can also be used to additional information such as whether an object such as a drainage bag is attached to the frame 12. For example, the drainage bag can be connected to a Foley catheter, which is used to drain urine from the bladder of a patient while resting on the patient support apparatus 10.

The patient support apparatus 10 further includes the load cells 76 which are communicatively connected to the computing device 702. The load cells 76 can communicate raw data to the computing device 702 that can be analyzed by the computing device 702 to detect the center of gravity or weight distribution of the patient while resting on the mattress 13 of the patient support apparatus 10, when the patient changes position or moves on the mattress 13 supported by the deck 14, or when the patient exits the patient support apparatus 10.

The patient support apparatus 10 further includes the mattress 13 which can include a pump 712 which can be controlled by the computing device 702 to inflate one or more bladders inside the mattress 13 to regulate the firmness of one or more portions of the mattress 13. In such examples, the mattress 13 is a powered air mattress that operates under different modes.

As an illustrative example, the mattress 13 can operate under a normal mode that provides continuous full-body pressure redistribution for the patient where the pump 712 is used to automatically adjust the bladders of the mattress 13 to accommodate changes in patient weight distribution. The mattress 13 can also operate under a maximum inflate mode in which the pump 712 inflates the bladders to maximize the firmness of the mattress 13. The mattress 13 can also operate under a turn mode where the pump 712 is used to inflate one side of the mattress 13 and deflate an opposite side of the mattress 13 to facilitate turning the patient on the mattress 13.

The mattress 13 can further includes pressure sensors 714 that measure the pressure inside the bladders of the mattress 13. Pressure changes inside the bladders of the mattress 13 detected by the pressure sensors 714 can be used to detect movement of the patient on the patient support apparatus 10, in addition to, or separately from, the data detected by the load cells 76.

As further shown in FIG. 7, the patient support apparatus 10 can include an antenna 716 that detects wireless signals from objects worn or carried by the users of the patient support apparatus 10 to identify users via proximity detection technology. The wireless signals detected by the antenna 716 can include radio-frequency identification (RFID) signals, near-field communication (NFC) signals, Wi-Fi signals, and/or Bluetooth^{®} signals. The wireless signal from each object carried by a user is unique for uniquely identifying each user. In some examples, the wireless signal is emitted by a tag attached to an object worn or carried by the user such as a badge, a lanyard, a name tag, and the like. In some examples, the wireless signal is emitted by a device 732 carried by the user such as a mobile smartphone, or the like.

In further examples, identification of the users of the patient support apparatus 10 can be accomplished by analyzing the visual data captured by the camera 90 (see FIG. 1). For example, facial recognition algorithms can be performed on the visual data captured by the camera 90 to determine an identity of a user of the patient support apparatus 10. Alternatively, or additionally, the visual data captured by the camera 90 can be analyzed to detect machine-readable data, a name, or other identification on an object worn by the user such as a badge, a lanyard, a name tag, and the like to determine the identity of the user.

In further examples, identification of the users of the patient support apparatus 10 can be accomplished by communicating with the EHR system 740 and/or the ADT system 750. For example, the EHR system 740 and/or the ADT system store data that identifies which caregivers are assigned to the patient or the room where the patient support apparatus 10 is located, which can be used for identifying the users of the patient support apparatus 10.

The patient support apparatus 10 includes a network interface 718 that allows the patient support apparatus 10 to connect to the network 720. The network interface 718 can include wired interfaces and/or wireless interfaces. For example, the network interface 718 can wirelessly connect to the network 720 such as through Wi-Fi and other wireless communications protocols. Alternatively, or additionally, the network interface 718 can connect to the network 720 using wired connections such as through Ethernet or Universal Serial Bus (USB) cables.

The network 720 can include any type of wired or wireless connections or any combinations thereof. Examples of wireless connections include Wi-Fi, Bluetooth, ultra-wideband (UWB), radio frequency identification (RFID), cellular network connections, and the like. In some examples, the network 720 is an Internet-of-things (IoT) network that connects and exchanges data between one or more devices and the patient support apparatus 10 over the Internet or other communications networks. In alternative examples, one or more of the devices can directly communicate with the patient support apparatus 10 without using the network 720 such as via direct wireless or wired connections with the patient support apparatus 10.

The nurse call system 730 facilitates efficient interaction between a patient resting on the patient support apparatus 10 and caregivers in the healthcare facility. The nurse call system 730 can include bedside call buttons on or adjacent to the patient support apparatus 10 that allow the patient while resting on the patient support apparatus 10 to alert nurses or other caregivers when they need assistance. Once activated, the nurse call system 730 sends a signal to the nurse's station or to devices 732 carried by the caregivers while on shift in the healthcare facility to notify the caregivers of the patient's request and their location. The nurse call system 730 ensures prompt responses to medical needs of the patient while resting on the patient support apparatus 10 to improve workflow efficiency and promote patient safety.

The EHR system 740 maintains the medical history of the patient by consolidating health information, including patient demographics, medical diagnoses, treatment plans, medications, test results, and immunization history, into a single electronic document called an electronic health record (EHR) 742. The EHR system 740 manages a plurality of EHRs 742 for a plurality of patients. The EHR system 740 enables sharing of data among healthcare professionals to improve coordination of care, reduce errors, and enhance patient outcomes. The EHR system 740 also supports clinical decision-making by providing real-time access to comprehensive patient information. The EHR system 740 can include features such as secure data storage and integration with other health technologies, which streamline administrative tasks, reduce paperwork, and improve overall healthcare efficiency and patient safety.

The ADT system 750 tracks and manages a patient's journey from admission to the healthcare facility through discharge or transfer to another facility. When a patient is first admitted, the ADT system 750 records details such as personal information, medical history, and the reason(s) for admission, while also assigning a unique identifier to the patient. When the patient is discharged or transferred, the ADT system 750 updates the status of the patient, ensures accurate billing, and facilitates the transfer of relevant medical records to other departments or facilities. By automating these processes, the ADT system 750 improves the efficiency of operations in the healthcare facility, reduces errors, ensures regulatory compliance, and enhances patient care by providing up-to-date information to healthcare providers.

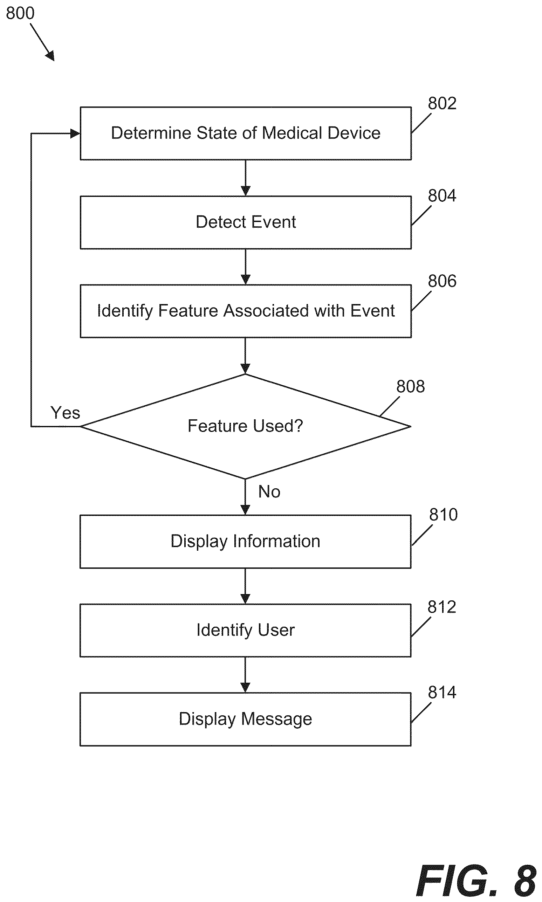

FIG. 8 schematically illustrates an example of a method 800 of providing medical device training. The method 800 can be performed by the Al application 708 which is executed by the computing device 702 of the patient support apparatus 10 to provide training recommendations to improve the effectiveness and efficiency of the patient support apparatus 10.

The method 800 includes an operation 802 of determining a state of a medical device. In examples where the medical device is the patient support apparatus 10, the state of the medical device can be determined based at least partially on the position of the frame 12. For example, operation 802 can include determining whether the patient support apparatus 10 is in the lower position (see FIG. 2), the upper position (see FIG. 3), the Trendelenburg position (see FIG. 4), or the reverse Trendelenburg position (see FIG. 5). The position of the frame 12 can be determined based on data received from the one or more sensors 710 of the actuator system 56 and/or based on visual data of the patient support apparatus 10 captured by the camera 90.

Additionally, operation 802 can include determining the state of the medical device based on the mode of the mattress 13 such as whether the mattress 13 is in the normal mode, the maximum inflate mode, the turn mode, or another mode of operation. The mode of the mattress 13 is determined based on the data received from the pressure sensors 714 of the mattress 13.

The method 800 includes an operation 804 of detecting an event that occurs during the state of the medical device determined in operation 802. Operations 802, 804 can occur simultaneously. In examples where the medical device is the patient support apparatus 10, operation 804 can include detecting the event based on the data from the load cells 76 on the frame 12 and/or data from the pressure sensors 714 of the mattress 13.

In some examples, operation 804 includes detecting the event based on the visual data captured by the camera 90. In such examples, the visual data can be analyzed using artificial intelligence to detect the event based on user interactions with the patient support apparatus 10.

Operation 804 can include detecting the event based on movement of the patient on the patient support apparatus 10. For example, operation 804 can include detecting the event when the patient is pulled up on the mattress 13, when the patient is turned on the mattress 13, when the patient is transferred from the patient support apparatus 10 to another patient support apparatus or surface, or when the patient is helped to exit the patient support apparatus 10.

Operation 804 can include using artificial intelligence to detect the event when a patient is pulled up on the mattress 13 by a caregiver based on the data from the load cells 76 of the frame 12 and/or the data from the pressure sensors 714 of the mattress 13.

Operation 804 can include using artificial intelligence to detect the event when a patient is turned on the mattress 13 by a caregiver based on the data from the load cells 76 of the frame 12 and/or the data from the pressure sensors 714 of the mattress 13.

Operation 804 can include using artificial intelligence to detect the event when a patient is laterally transferred from the patient support apparatus 10 to another patient support apparatus or other type of support surface based on the data from the load cells 76 of the frame 12 and/or the data from the pressure sensors 714 of the mattress 13.

Operation 804 can include using artificial intelligence to detect an event when a patient is being helped to exit the patient support apparatus 10 based on the data from the load cells 76 of the frame 12 and/or the data from the pressure sensors 714 of the mattress 13.

Operation 804 can include detecting a drainage bag is attached to the frame 12. In such examples, the drainage bag can be connected to a Foley catheter, which is a tube that helps to drain urine from the bladder of the patient. The attachment of the drainage bag can be detected based on data from one or more sensors on the frame 12 of the patient support apparatus 10 such as the sensors 710. Alternatively, or additionally, operation 804 can include the patient support apparatus 10 connecting to the EHR system 740 to determine whether a foley catheter has been prescribed to the patient assigned to the patient support apparatus 10.

In some examples, operation 804 can include determining one or more aspects of the patient occupying the patient support apparatus 10 such as whether the patient has an above average height or is a falls risk. In such examples, the one or more aspects of the patient can be determined based on the data stored in the EHR 742 of the patient. As shown in FIG. 7, the patient support apparatus 10 is communicatively connected to the EHR system 740 such that the patient support apparatus 10 has access to the EHR 742 of the patient.

As shown in FIG. 8, the method 800 includes an operation 806 of identifying a feature of the medical device associated with the event detected in operation 804. In examples where the medical device is the patient support apparatus 10, operation 806 can include identifying one or more features of the patient support apparatus 10 that can improve performance of the event detected on the patient support apparatus 10 such as pulling up the patient on the mattress 13, turning the patient on the mattress 13, laterally transferring the patient to another patient support apparatus, and other types of events and actions.

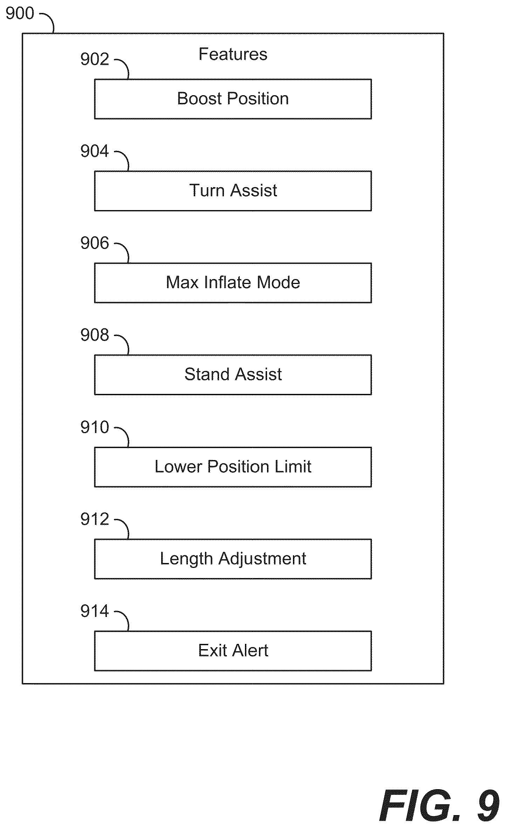

FIG. 9 illustrates examples of features of the patient support apparatus 10 that can be used to improve performance of an event performed the patient support apparatus 10. The features shown in FIG. 9 are not exhaustive such that the patient support apparatus 10 can include additional features that are not illustrated. In some examples, the patient support apparatus 10 can include fewer features than the features shown in FIG. 9.

As shown in FIG. 9, the patient support apparatus 10 can include a boost position feature 902 that causes the actuator system 56 to level the head section 22 and the foot section 28 of the deck 14 and to position the deck 14 into Trendelenburg position (see FIG. 4). In some examples, the boost position feature 902 can also cause the pump 712 to inflate the mattress 13 to have the maximum inflate mode. These adjustments can help a caregiver to reposition the patient toward the head end of the mattress 13 (i.e., to pull up the patient on the mattress 13).

The patient support apparatus 10 can include a turn assist feature 904 that causes the pump 712 to inflate bladders on one side of the mattress 13 and to deflate bladders on an opposite side of the mattress 13. These adjustments to the mattress 13 can help a caregiver to turn the patient from left to right or from right to left on the mattress 13.

The patient support apparatus 10 can include a maximum inflate mode 906 that causes the pump 712 to inflate bladders of the mattress 13 to maximizes the firmness of the mattress 13. The maximum inflate mode 906 can assist caregivers to laterally transfer the patient to another support surface and/or to adjust the patient's position on the mattress 13.

The patient support apparatus 10 can include a stand assist mode 908 that causes the actuator system 56 to raise the head section 22 and lower the seat section 26 of the deck 14 to help the patient more easily exit the patient support apparatus 10. In some examples, the stand assist mode 908 can further cause the pump 712 to inflate the bladders of the mattress 13 adjacent to where the seat section 26 of the deck 14 is located to provide additional support.

The patient support apparatus 10 can include a lower position limit feature 910 that restricts how much the foot section 28 of the deck 14 can be lowered relative to the ground. The lower position limit feature 910 can reduce the likelihood of a drainage bag touching the floor which can cause contamination of the drainage bag and lead to an infection of the patient.

The patient support apparatus 10 can include a length adjustment feature 912 that causes the actuator system 56 to extend or retract the foot section 28 of the deck 14 to accommodate patients who have various heights. During the adjustment, the foot section 28 can be stopped at any distance between a retracted position and a fully extended position.

The patient support apparatus 10 can include an exit alert mode 914 that triggers an alert that can be communicated to the one or more devices 732 associated with caregivers in the healthcare facility. The exit alert mode 914 can have different levels of sensitivity settings such as a position mode that alerts when the patient changes position on the patient support apparatus 10 to notify the caregivers when the patient begins to move. The exit alert mode 914 can have an exiting mode that alerts when the patient moves away from the center and towards the edge of the mattress 13 to notify the caregivers when a potential exit is attempted. The exit alert mode 914 can also have an out of bed mode that alerts when the patient's weight has left the patient support apparatus to notify the caregivers when the patient leaves the patient support apparatus.

The method 800 includes an operation 808 of determining whether the feature of the medical device identified in operation 806 was used during the event detected in operation 804. When the feature identified in operation 806 was used during the event detected in operation 804 (i.e., "Yes" in operation 808), the method 800 returns to operation 802 to continue monitoring the state of the medical device and to continue detecting events that occur on the medical device.

As shown in FIG. 8, when the feature identified in operation 806 was not used during the event detected in operation 804 (i.e., "No" in operation 808), the method 800 proceeds to an operation 810 of displaying information regarding a recommendation about utilizing the feature when the state of the medical device indicates the feature was not used when the event occurred.

In examples where the medical device is the patient support apparatus 10, operation 808 can include determining whether the boost position feature 902 was used when operation 804 includes detecting the patient being pulled up the mattress 13. Operation 808 can include determining whether the turn assist feature 904 was used when operation 804 includes detecting the patient being turned on the mattress 13. Operation 808 can include determining whether the maximum inflate mode 906 was used when operation 804 includes detecting the patient as being laterally transferred to another patient support apparatus or support surface. Operation 808 can include determining whether the stand assist mode 908 was used when operation 804 includes detecting the patient as being helped to exit the patient support apparatus 10.

Operation 808 can also include determining whether the lower position limit feature 910 was used when operation 804 includes detecting a drainage bag attached to the patient support apparatus 10. Operation 808 can also include determining whether the length adjustment feature 912 was used when operation 804 includes detecting the patient occupying the patient support apparatus 10 as having an above average height. Operation 808 can further include determining whether the exit alert mode 914 was used when operation 804 includes detecting the patient occupying the patient support apparatus 10 as having a falls risk. Additional examples are contemplated based on the features of the patient support apparatus 10.

Operation 810 can include recommending training for any one of the boost position feature 902, the turn assist feature 904, the maximum inflate mode 906, the stand assist mode 908, the lower position limit feature 910, the length adjustment feature 912, the exit alert mode 914, or additional features and/or modes available on the patient support apparatus 10. As an illustrative example, operation 810 can include displaying information on the display screen 25 such as "It appears the patient has been pulled up in bed, but the Boost feature was not used. Would you like more information about the Boost feature?"

In alternative examples of the method 800, operation 804 can include tracking a frequency of using the features of the medical device over a predetermined period of time and/or for a given number of patients. When operation 808 determines that the usage frequency for one or more features of the medical device is less than an expected frequency for the features, operation 810 includes recommending training for the one or more features.

In some examples, the method 800 can include an operation 812 of identifying a user of the medical device when the event occurs. For example, operation 812 can include using the antenna 716 to detect a wireless signal emitted from the another object carried by the user, and determining the identity of the user based on the wireless signal. Alternatively, operation 812 can include analyzing the visual data captured by the camera 90 to determine the identity of the user such as by performing facial recognition, or reading a machine-readable label, a name, or other identification on an object worn by the user such as a badge, a lanyard, a name tag, and the like. In yet further examples, operation 812 can include retrieving data from the EHR system 740 and/or the ADT system 750 to determine the identity of the user of the patient support apparatus.

The method 800 further includes an operation 814 of displaying a message that includes the training recommended in operation 810. In some examples, operation 814 includes displaying the message with the training recommendation on a display screen of the medical device. In examples where medical device is the patient support apparatus 10, operation 814 can include displaying the message on the display screen 25 of the patient support apparatus 10.

FIG. 10 illustrates an example of the display screen 25 of the patient support apparatus 10 that includes a message 1000 displayed in accordance with operation 814 of the method 800. In this illustrative example, the message 1000 recommends training for the boost position feature 902 (e.g., "BOOST Button can assist in pulling a patient up in bed. Would you like to learn about BOOST?"). The message 1000 includes a first option 1002 to accept the training and a second option 1004 to decline the training. Selection of the first option 1002 causes a tutorial, a video, or other media to be displayed on the display screen 25. Selection of the second option 1004 causes the message 1000 to disappear. The message 1000 further includes machine-readable data 1006 that when scanned by a camera of a mobile device (e.g., the devices 732 carried by the caregivers) causes the training to be displayed on the mobile device.

In examples where an identity of the user is identified in operation 812, operation 814 can include displaying the message on a display screen of another device of the user. For example, the nurse call system 730 can route the message for display on a device 732 based on the identity of the user detected in operation 812. In such examples, the message is targeted to a particular user of the medical device to provided targeted training of the medical device.

In view of the foregoing, a medical device such as the patient support apparatus 10 automatically detects when a feature that would be helpful during a particular context is not used for the provision of healthcare to a patient, and offers training for the feature.

Embodiments of the invention can be described with reference to the following numbered clauses, with preferred features laid out in the dependent clauses:
1. A patient support apparatus, comprising:
   a frame;
   a deck coupled to the frame, the deck being movable relative to the frame, and the deck being configured to support a mattress;
   at least one actuator configured to move at least one of the mattress and the deck to perform a therapy or caregiver assistance function;
   at least one sensor configured to detect movement of a patient supported on the mattress;
   at least one processing device; and
   at least one memory device storing software instructions that, when executed by the at least one processing device, cause the at least one processing device to:
      detect a state of actuation of the at least one actuator;
      determine a type of movement of the patient based on one or more outputs of the at least one sensor;
      identify an actuation feature of the at least one actuator that is associated with the type of movement of the patient;
      determine whether the actuation feature is utilized when the type of movement of the patient occurred based on the state of actuation of the at least one actuator; and
      recommend information about utilizing the actuation feature when the actuation feature is not utilized when the type of movement of the patient occurred.
2. The patient support apparatus of clause 1, wherein the movement type of the patient includes at least one of a patient turn, a patient transfer, and a patient pull up.
3. The patient support apparatus of clause 2, wherein the actuation feature includes at least one of a turn assistance feature, a maximum inflation feature, and a patient boost feature.
4. The patient support apparatus of any preceding clause, wherein the at least one sensor includes one or more pressure sensors that measure pressures inside one or more bladders of the mattress.
5. The patient support apparatus of clause 4, wherein the at least one actuator includes a pump to inflate the one or more bladders of the mattress.

## Claims

1. A system for operating a medical device, the system comprising:
at least one processing device; and
at least one memory device storing software instructions that, when executed by the at least one processing device, cause the at least one processing device to:
determine a state of the medical device;
detect an event that occurs during the state of the medical device;
identify a feature of the medical device that is associated with the event; and
display information regarding a recommendation about utilizing the feature when the state of the medical device indicates the feature was not used when the event occurred.

2. The system of claim 1, wherein the instructions, when executed by the at least one processing device, further cause the at least one processing device to:
display a message on a display screen of the medical device, wherein the message includes a first option to accept the training and a second option to decline the training.

3. The system of claim 2, wherein the message further includes machine-readable data that when scanned by a mobile device causes the training to be displayed on the mobile device.

4. The system of any preceding claim, wherein the instructions, when executed by the at least one processing device, further cause the at least one processing device to:
identify a user of the medical device when the event occurs;
generate a message that includes the training; and
transmit the message to another device associated with the user of the medical device.

5. The system of any preceding claim, wherein the medical device is a patient support apparatus that includes:
a frame;
load cells positioned on the frame for detecting movement of a patient occupying the patient support apparatus, wherein the event is detected based on the movement of the patient;
an actuator system for adjusting a position of the frame;
one or more sensors for detecting the position of the frame, wherein the state of the medical device is determined based at least partially on the position of the frame; and
a deck coupled to the frame, the deck configured to support a mattress.

6. The system of claim 5, wherein the instructions, when executed by the at least one processing device, further cause the at least one processing device to:
detect the event as a patient being pulled up the mattress;
determine the state of the medical device is not using a boost position feature, wherein the boost position feature causes the actuator system to level a head section and a foot section of the deck and move the deck into a Trendelenburg position; and
recommend the training for the boost position feature.

7. The system of either claim 5 or claim 6, wherein the instructions, when executed by the at least one processing device, further cause the at least one processing device to:
detect the event as a patient turning on the mattress;
determine the state of the medical device is not using a turn assist feature, wherein the mattress includes bladders that are inflatable, and wherein the turn assist feature causes one side of the mattress to deflate and an opposite side of the mattress to inflate; and
recommend the training for the turn assist feature.

8. The system of any one of claims 5 to 7, wherein the instructions, when executed by the at least one processing device, further cause the at least one processing device to:
detect the event as a patient transferring to another patient support apparatus;
determine the state of the medical device is not using a maximum inflate mode, wherein the mattress includes bladders that are inflatable to adjust a firmness of the mattress, and wherein the maximum inflate mode causes the mattress to inflate to a maximum firmness; and
recommend the training for the maximum inflate mode.

9. The system of any one of claims 5 to 8, wherein the instructions, when executed by the at least one processing device, further cause the at least one processing device to:
detect the event as a patient exiting the patient support apparatus;
determine the state of the medical device is not using a stand assist mode, wherein the stand assist mode causes the actuator system to raise a head section of the deck and lower a seat section of the deck to facilitate exiting the patient support apparatus; and
recommend the training for the stand assist mode.

10. The system of any one of claims 5 to 9, wherein the instructions, when executed by the at least one processing device, further cause the at least one processing device to:
detect a drainage bag is attached to the patient support apparatus;
determine the state of the medical device is not using a lower position limit feature, wherein the lower position limit feature restricts lowering a foot section of the deck to prevent contamination of the drainage bag; and
recommend the training for the lower position limit feature.

11. The system of any one of claims 5 to 10, wherein the instructions, when executed by the at least one processing device, further cause the at least one processing device to:
determine a patient occupying the patient support apparatus has an above average height;
determine the state of the medical device is not using a length adjustment feature that causes the actuator system to adjust a length of the deck; and
recommend the training for the length adjustment feature.

12. The system of any one of claims 5 to **11,** wherein the instructions, when executed by the at least one processing device, further cause the at least one processing device to:
determine a patient occupying the patient support apparatus is a falls risk;
determine the state of the medical device is not using an exit alert mode, wherein the exit alert mode triggers an alert when the patient exits the patient support apparatus; and
recommend the training for the exit alert mode.

13. A method for operating a medical device, the method comprising:
determining a state of the medical device;
detecting an event that occurs during the state of the medical device;
identifying a feature of the medical device that is associated with the event; and
displaying training for the feature when the state of the medical device indicates the feature was not used when the event occurred.

14. The method of claim 13, further comprising:
identifying a user of the medical device when the event occurs;
generating a message that includes the training; and
transmitting the message to the mobile device associated with the user.

15. The method of either claim 13 or claim 14, wherein the training is for at least one of a boost position feature, a turn assist feature, a maximum inflate mode, a stand assist mode, a lower position limit feature, a length adjustment feature, and an exit alert mode for a patient support apparatus.
